(19)

**Europäisches Patentamt**
**European Patent Office**
**Office européen des brevets**

(11) **EP 3 725 224 B1**

(12) **EUROPEAN PATENT SPECIFICATION**

(45) Date of publication and mention
of the grant of the patent:
**28.05.2025 Bulletin 2025/22**

(21) Application number: **19169542.8**

(22) Date of filing: **16.04.2019**

(51) International Patent Classification (IPC):
**A61B 5/107** (2006.01)   **A61B 5/00** (2006.01)
**G06T 7/00** (2017.01)   **G06T 7/30** (2017.01)

(52) Cooperative Patent Classification (CPC):
**A61B 5/1073; A61B 5/0042; A61B 5/4064;
G06T 7/0016; G06T 7/30;** A61B 2576/026;
G06T 2207/10088; G06T 2207/30016

(54) **METHOD AND SYSTEM FOR DETERMINING THE RATE OF CHANGE OF A QUANTITATIVE PARAMETER**

VERFAHREN UND SYSTEM ZUR BESTIMMUNG DER ÄNDERUNGSRATE EINES QUANTITATIVEN PARAMETERS

PROCÉDÉ ET SYSTÈME POUR DÉTERMINER LA VITESSE DE CHANGEMENT D'UN PARAMÈTRE QUANTITATIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.10.2020 Bulletin 2020/43**

(73) Proprietors:
• **Siemens Healthineers AG**
**91301 Forchheim (DE)**
• **Centre Hospitalier Universitaire Vaudois**
**1011 Lausanne (CH)**

(72) Inventors:
• **RICHIARDI, Jonas**
**1202 Genève (CH)**
• **MARÉCHAL, Bénédicte**
**1003 Lausanne (CH)**
• **KOBER, Tobias**
**1007 Lausanne (CH)**
• **CORREDOR JEREZ, Ricardo**
**1018 Lausanne (CH)**

(74) Representative: **Fischer, Michael et al**
**Siemens AG**
**Postfach 22 16 34**
**80506 München (DE)**

(56) References cited:
**WO-A1-2015/066052    US-A1- 2016 051 178**

• **BLAKE C. JONES ET AL: "Quantification of multiple-sclerosis-related brain atrophy in two heterogeneous MRI datasets using mixed-effects modeling", NEUROIMAGE: CLINICAL, vol. 3, 13 August 2013 (2013-08-13), pages 171 - 179, XP055627355, ISSN: 2213-1582, DOI: 10.1016/ j.nicl.2013.08.001**
• **OLGA VOEVODSKAYA ET AL: "The effects of intracranial volume adjustment approaches on multiple regional MRI volumes in healthy aging and Alzheimer's disease", FRONTIERS IN AGING NEUROSCIENCE, vol. 6, 7 October 2014 (2014-10-07), XP055626835, DOI: 10.3389/ fnagi.2014.00264**
• **MAHESWARAN S ET AL: "Longitudinal regional brain volume changes quantified in normal aging and Alzheimer's APPxPS1 mice using MRI", BRAIN RESEARCH, ELSEVIER, AMSTERDAM, NL, vol. 1270, 13 May 2009 (2009-05-13), pages 19 - 32, XP026085013, ISSN: 0006-8993, [retrieved on 20090309], DOI: 10.1016/ J.BRAINRES.2009.02.045**
• **HUA XUE ET AL: "MRI-based brain atrophy rates in ADNI phase 2: acceleration and enrichment considerations for clinical trials", NEUROBIOLOGY OF AGING, TARRYTOWN, NY, US, vol. 37, 19 October 2015 (2015-10-19), pages 26 - 37, XP029344322, ISSN: 0197-4580, DOI: 10.1016/J.NEUROBIOLAGING.2015.09.018**

**(Cont. next page)**

EP 3 725 224 B1

- MARK FIECAS ET AL: "The generalized shrinkage estimator for the analysis of functional connectivity of brain signals", ARXIV.ORG, CORNELL UNIVERSITY LIBRARY, 201 OLIN LIBRARY CORNELL UNIVERSITY ITHACA, NY 14853, 16 August 2011 (2011-08-16), XP080521134, DOI: 10.1214/10-AOAS396

**Description**

Field of the invention

The present disclosure is directed, in general, to imaging techniques for imaging biological objects, like tissues, and more specifically to techniques for measuring changes in quantitative parameters characterizing a biological object, such as a brain volume change using Magnetic Resonance Imaging (MRI) .

Background

Measuring changes of quantitative parameters over time for biological parameters of a biological object is important for characterizing the biological evolution of such biological object. For instance, measuring a change within the volume of a brain region is important for physicians to evaluate brain pathological processes. This is of particular relevance in the current context of an aging population worldwide which will stress the resources of public health systems. Indeed, dementia, including Alzheimer's disease (AD), affects around 10 million new people every year, and is one of the most prevalent old age diseases. A classical warning sign of Alzheimer's disease is a reduction of the volume of the brain or sub-regions of the brain, called brain atrophy, and resulting from pathological processes including neuro-fibrillary tangles caused by Tau protein degeneration. Another prevalent disease where atrophy can serve as a useful marker of disease progression is Multiple sclerosis, where brain atrophy is now part of the so-called NEDA-4 ("No Evidence of Disease Activity") criteria guiding treatment decisions. Beyond these concrete examples, many other biomarkers in the medical realm are followed up one or more time to either observe the activity of a disease or the effect of an administered treatment.

Atrophy can be observed in-vivo using MRI. However, such changes are initially subtle (about -0.5% to -0.2% annual atrophy rate in healthy controls depending on the brain region with only subtle increase of that rate in patients) and very difficult to detect automatically over short periods due to measurement noise and algorithmic instability. It is obvious that the closer in time two consecutive noisy measurement points are, the bigger the potential error becomes. Indeed, for many brain structures, the biological annual atrophy rate is smaller than apparent changes that come from repeated measurements of the same subject in back-to-back sessions. Thus, actual change is buried in measurement noise induced by the employed hardware, image analysis software, preprocessing or image acquisition steps. Examples include the use of different imaging hardware (the scanner itself or e.g. used MR coils), different scanner versions, different calibration, acquisition parameters or protocols. This is particularly problematic when the goal is to establish reference ranges for normal atrophy, because the distribution of atrophy rates for healthy subjects and patients shows large overlap due to this estimation error.

One obvious solution is to observe the patient for a long period (several years) and to obtain several measurements. As the effects of pathological changes are typically increasing over time, this can lead to more reliable estimates of atrophy rates, and reliable reference ranges. Unfortunately, an accurate quantification of brain atrophy is made difficult by changes in the parameters of MRI acquisition, coming notably from the evolution of MRI techniques over time. In order to solve this issue, Blake C. Jones et al. ("Quantification of multiple-sclerosis-related brain atrophy in two heterogeneous MRI datasets using mixed-effects modeling", NeuroImage: Clinical 3: 171-179, 2013) proposes a method for coherently estimating brain atrophy rates in heterogeneous MS sample via linear mixed effects multivariable regression incorporating three critical assumptions: 1) using age at time of scanning, rather than time since baseline, as the regressor of interest; 2) scanning individuals with a variety of techniques; and (3) introducing a simple additive correction for major differences in MRI protocols. However, in clinical practice, acquiring more images and waiting for the disease to progress is not practical or clinically very useful and might impede timely treatment - one would like to detect abnormal atrophy as rapidly as possible.

There are currently two main families of techniques to compute atrophy between baseline and follow-up images:

- a first family of techniques related to global image deformations between image pairs, and
- a second family of techniques related to regional brain volume computations on each individual image followed by statistical modelling. The present invention belongs to the second family.

The first family of techniques such as the technique disclosed in Teipel et al. (Multivariate deformation-based analysis of brain atrophy to predict Alzheimer's disease in mild cognitive impairment, NeuroImage, 2007) relies on "warping" or modifying each subject's image so that it matches an age-appropriate template as much as possible. Then, the amount of warping (the so-called "Jacobian of the deformation field") is taken as a measure of local deformation which represents atrophy. One example of using this technique longitudinally is found in the context of Multiple Sclerosis (MS) where a baseline image might be registered to a follow-up image for generating a deformation field DF1 and the follow-up image might be registered to the baseline image for generating a deformation field DF2. Then, the determinants of the Jacobians of DF1 and DF2 represent local volume change, which are then summed regionally to yield atrophy (or hypertrophy) estimates.

The second family of techniques comprises two main techniques:

- a first main technique using a short-term estimate to build a reference range (see for instance Ledig et al., Structural brain imaging in Alzheimer's disease and mild cognitive impairment: biomarker analysis and shared morphometry database, Sci. Rep. 8 (2018)); and
- a second main technique using an increase of the scanning frequency over short intervals (see for instance Schott et al., Combining short interval MRI in Alzheimer's disease, J. Neurol. 253, 1147-1153 (2006)).

Both approaches have very significant shortcomings: According to the first main technique, a baseline scan and a follow-up scan at 12 or 24 months are used to compute the atrophy rate for all subjects of a cohort, and the distribution of such rates for healthy controls is taken as the reference range for atrophy. While this allows discrimination of clinical groups, the obtained estimate has a high standard deviation (e.g. for the Hippocampus, healthy controls are estimated to have a mean atrophy rate of -1.1% /year with a standard deviation of 1.7% / year) and is biased with respect to the actual rate. According to the second main technique, patients are scanned every 2-3 months (up to 10 scans over 2 years).

While the second main technique indeed provides better estimates of atrophy, it implies significantly increased scanner use and hence potentially unacceptable healthcare costs.

Summary of the invention

It is an objective of the present invention, as defined by the appended claims, to propose a method and a system capable of providing reliable measurements of a quantitative parameter change rate for a biological object (for instance a volume change rate of a brain region, like an atrophy rate) when considering a biological process which takes place over a long period of time, wherein said change rate of the quantitative parameter is determined from a small number of quantitative parameter measurements (for instance, a small number of scans comprising ideally maximum two scans) over a short period of time with respect to the biological process (in the example given by Alzheimer's-induced atrophy typically between 8 and 16 months, ideally 12 months).

Said objective is achieved according to the present invention by a method and a system for determining a quantitative parameter change rate, such as a brain volume change rate, according to the object of the independent claims. Dependent claims present further advantages of the invention.

The present invention proposes a method for measuring within a short period of time a change rate of a quantitative parameter for a biological object of a subject, wherein said quantitative parameter enables to characterize a biological process that takes place over a long period of time, said method comprising:

- measuring said quantitative parameter at a first time T1 and at a second time T2 within said short period of time (short with respect to the measurement noise in each time point), wherein the value of the quantitative parameter measured at the first time T1 is denoted Q1 and the value of the quantitative parameter measured at the second time T2 is denoted Q2;
- determining an approximative change rate r of the measured quantitative parameter in function of Q2, Q1 and the interval of time d separating the measurement of Q1 from the measurement of Q2, e.g. r = 365*(Q2-Q1)/(d*Q1);

the method being characterized in that the system according to the invention automatically determines a regularized $r_{reg}$ estimate of the change rate using the equation:

$$r_{reg} = (1-\lambda)r + \lambda m \quad (1)$$

wherein

m is a mean value for said change rate of the quantitative parameter obtained by applying a reference data model to values of said quantitative parameters obtained by measurements of the latter for said biological object of healthy subjects, wherein for each healthy subject, values of the quantitative parameter are obtained by making a series of said measurements over a long period of time (i.e. the time difference between the first measurement and the last measurement of said quantitative parameter is more or less equal to said long period of time and is in any case greater than said short period of time); and $\lambda$ is computed analytically using an estimate of the variance of the values of the quantitative parameters obtained for said healthy subjects.

In particular, in the case of MRI, the present invention proposes to use infrequent scanning, namely a single baseline image and a single follow-up image for measuring the change rate of a brain region volume, wherein the reference data model is used for reducing the measurement noise. Advantageously, and contrary to prior art techniques, the measurement error obtained according to the proposed method can be lower than the volume change rate that needs to be estimated, providing therefore a more robust volume change rate measurement. According to the present invention, the reference data model is configured for restraining a calculation of the volume change rate, e.g. atrophy rate, based on expected rates within a comparable cohort. Advantageously, the present invention makes also possible a clinical applicability of automated brain morphometry for atrophy estimation, resulting thus in a particularly valuable tool for dementia estimation by physicians.

For instance, in the case of AD, the present invention proposes more specifically to leverage low-bias estimates of atrophy obtained on a large reference sample from the healthy subjects, i.e. create a statistical model - said reference data model - based on existing data to reduce the noise in the morphometric measurements of a patient, and to mathematically combine long-term, low-bias estimates of atrophy obtained on a reference sample with short-term, high-bias estimates obtained on individual patients.

The present invention proposes also a system for carrying out the previously described method.

The foregoing has outlined rather broadly the features and technical advantages of the present disclosure so that those skilled in the art may better understand the detailed description that follows. In particular, the present invention may help a physician to diagnose a disease for a biological object, which is typically an organ, like a brain.

Additional features and advantages of the disclosure will be described hereinafter that form the object of the claims. Those skilled in the art will appreciate that they may readily use the concept and the specific embodiment disclosed as a basis for modifying or designing other structures for carrying out the same purposes of the present disclosure. Those skilled in the art will also realize that such equivalent constructions do not depart from the scope of the disclosure in its broadest form.

For a more complete understanding of the present disclosure, and the advantages thereof, reference is now made to the following descriptions taken in conjunction with the accompanying drawings, wherein like numbers designate like objects, and in which:

Brief description of the drawings

Figure 1 illustrates a flowchart of a method for quantitative parameter change rate measurement according to the invention;

Figure 2 illustrates a system for implementing the claimed method.

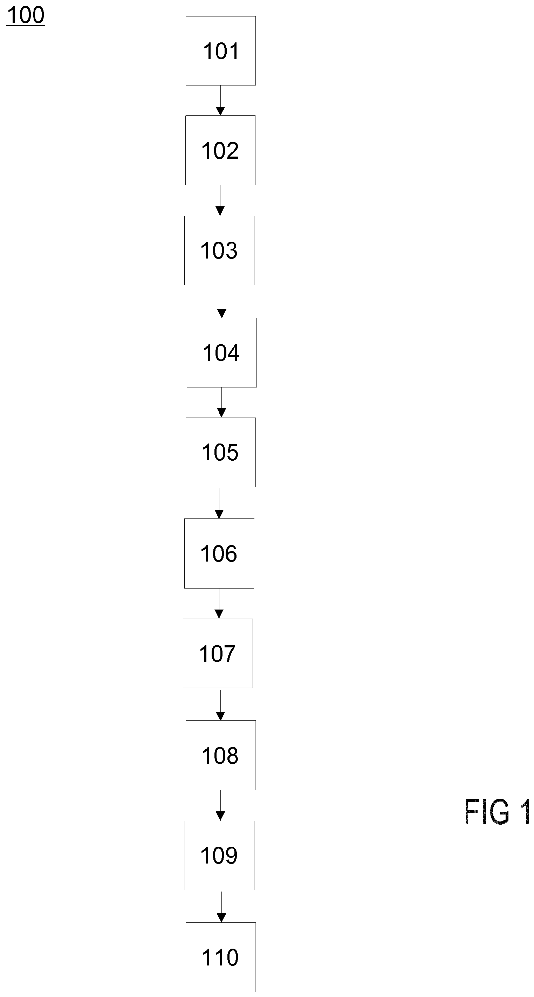

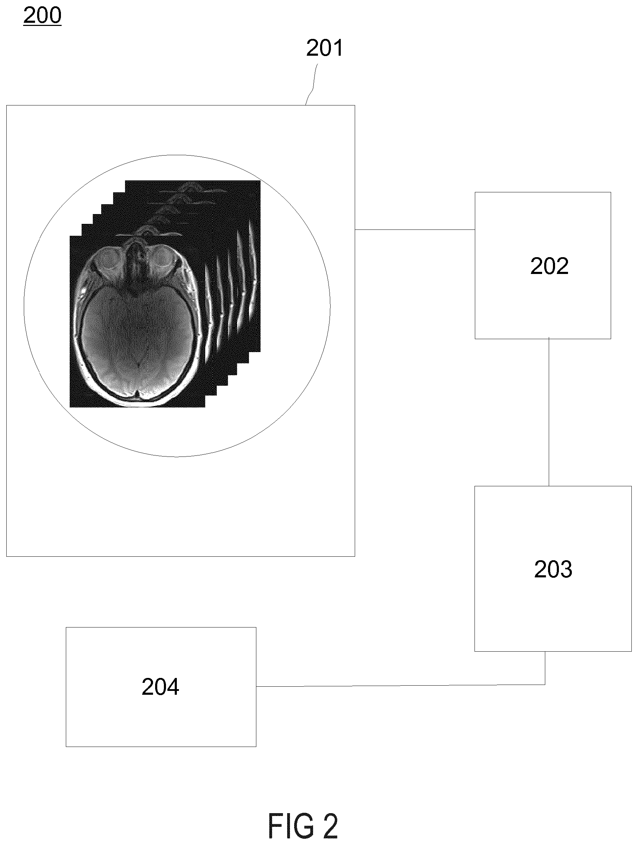

Figures 1 and 2, discussed below, and the various embodiments used to describe the principles of the present disclosure in this patent document are by way of illustration only and should not be construed in any way to limit the scope of the disclosure. Those skilled in the art will understand that the principles of the present disclosure may be implemented in any suitably arranged device. The numerous innovative teachings of the present application will be described with reference to exemplary non-limiting embodiments.

Detailed description

We will now describe in more details the method according to the invention through Figure 1 which describes the different steps of the method 100 carried out by the system according to the invention for determining a quantitative parameter change rate for a biological object of a new subject. The method according to the invention comprises notably two essential elements: (i) the use of a reference data model describing the longitudinal course of the measurements, i.e. a statistical formula or an algorithm (which could also be a neural network), and (ii) longitudinal measurements of the quantitative parameter to parametrize the reference data model so that it "learns" the expected longitudinal course ( for instance an atrophy rate). In the preferred embodiment described below, said biological object is a brain and said quantitative parameter is the volume of the brain or of a brain region, which can be measured for instance using MRI techniques. It must however be noted that the present concept might be used for determining the change rate of any quantitative parameter whose measurement over a short-time period is not reliable enough.

The method 100 comprises the following successive steps, which are preferentially automatically performed by the system according to the invention:

At step 101, the system according to the invention acquires or obtains brain images for each subject of a predefined population, wherein, for each of said subjects, brain images are obtained at different times (i.e. for different subject ages) for enabling a measurement of a brain volume change in function of time. In other words, the system acquires longitudinal brain images for measuring a longitudinal brain volume change. For instance, 8-10 longitudinal acquisitions of brain images are registered for each subject. Typically, in case of measurement of a brain atrophy rate, T1-weighted images are acquired or obtained for each subject of the predefined population using for instance an MPRAGE MRI acquisition. The predefined population comprises at least one set of healthy subjects, called hereafter healthy controls. In the case of AD, the predefined population may for instance comprise a first set of healthy controls, a second set of subjects with mild cognitive impairment, and a third set of subjects suffering from AD. Each acquired or obtained brain image is preferentially uniformly preprocessed by the system according to the invention (i.e. all images underwent the same distortion correction, bias field correction, etc.).

At step 102, the system according to the invention registers the images of each subject to their first time point (i.e. to the first longitudinal image acquired for the considered subject) using preferentially an affine registration.

At step 103, each brain image of each subject is segmented into different brain regions by the system. In other words, the system is configured for delineating said different brain regions in order to determine a set of brain region volumes (i.e. the volume of the hippocampus, a brain structure typically affected by Alzheimer's) of each subject.

At step 104, a reference data model of longitudinal brain volume change is trained on the longitudinal brain region volumes obtained for the set of healthy controls. Preferentially, the reference data model is a mixed effects model. It has indeed to be noticed that due to the clustering of data by subjects, an ordinary least-squares regression would be invalid. Training the reference data model on the volume change data obtained from the healthy controls enables to determine a mean volume change rate for said set of healthy controls and for each of said different brain regions enabling therefore to find different rates for different brain regions (also usually called "brain sub-regions"). In case of AD characterized by brain region atrophy, the mixed effects model is for instance a log-linear model of volume distributions that is modelled or represented as a function of a constant (see the constant taken equal to 1 in Eq. (2) below), a fixed effect for age (see "Age" in Eq. (2) below), a fixed effect for sex (see "Sex" in Eq. (2) below), and a random intercept (i.e. each subject can have different starting volumes) and random slope (i.e. each subject can have a different slope or rate of atrophy) for each subject. This corresponds to the following equation for each particular brain region:

$$\log(\text{volume}) \sim 1 + \text{Age} + \text{Sex} + (1 + \text{Age} \mid \text{Subject}) \quad (2)$$

wherein

volume is the brain region volume computed by step 103; Age is the age of the subject at the time of the scan configured for acquiring the subject brain image;
Sex is the biological sex of subject;
Subject is a categorical variable (factor) uniquely identifying the subject in the reference dataset. The parentheses and straight line "|" on the right-hand side are notational convention used in several mixed model software packages to indicate random effects, in this case there is a subject random effect.

The reference data model is trained on the set of healthy controls. For illustration purpose, the reference data model has been used in the case wherein the quantitative parameter is a brain region volume. Nevertheless, said reference data model might be used for other kinds of quantitative parameters and is therefore not restricted to the volume as single possible quantitative parameter. For said other kind of quantitative parameters, the above-mentioned explanations and description applies mutatis mutandis.

By applying the reference data model to the volume change data coming from the healthy controls, a volume change rate is therefore automatically determined by the system according to the invention for each of said different brain regions. For instance, by fitting with Eq. (2) the longitudinal brain region volumes measured and determined for the healthy controls, a longitudinal volume change rate, corresponding for instance to a "long-term" atrophy rate, in percent change per year can be directly obtained by the system according to the invention, said longitudinal volume change rate being a fitting coefficient m for the "Age" of the reference data model. The wording "long-term" is used for distinguishing the change rate obtained from applying the reference data model to longitudinal measurements that took place during a long period for each subject of the healthy controls (e.g. volume change data comprising more than one year of follow-up for each healthy subject and including several follow-up brain volume measurements) from the change rate obtained from at least two measurements taking place during a short period of time, said short period of time resulting in an important error on the value obtained for said change rate.

Preferentially, to avoid biasing results due to potentially restrictive parametric assumptions, the system is configured for using a parametric bootstrap with resamplings (typically, with hundreds of resamplings, e.g. 200 resamplings) to establish 5th/95th percentiles around the Age coefficient m, yielding a reference range for the long-term atrophy rate. Therefore, applying the reference data model to the healthy controls provides essential information with respect to volume change rate in a healthy aging sample. Preferentially, all or part of the volume change rates obtained from the healthy controls are stored in a database or memory of the system according to the invention for each of the brain regions.

At step 105, the system acquires or obtains a baseline brain image and a single follow-up brain image for the new subject, wherein the time interval d separating the acquisition of the baseline brain image and the single follow-up brain image is predefined and shorter than the time period during which the longitudinal acquisition of brain images took place for the healthy controls. Indeed, said time interval defines preferentially short-term measurements, i.e. said time interval d between the acquisition of the baseline brain image and the follow-up brain image is typically several months, for instance 8-16 months, preferentially 12 months.

At step 106, the system may register the follow-up brain image onto the baseline brain image using preferentially an affine registration.

At step 107, the baseline brain image and the follow-up brain image are segmented into said different brain regions by the system, as described in step 103, so that the same set of brain region volumes is obtained.

At step 108, the system determines the volume change rate of the new subject from said baseline and follow-up brain images for at least one and preferentially each of said different brain regions, wherein for the concerned brain region, a relative percent volume change p is computed by the system using

$$p = (Vnew - Vold) / Vold \quad (3),$$

and a "short-term" approximative volume change rate r is computed using

$$r = p/d * 365 \quad (4),$$

wherein the time interval d is expressed in days, Vnew is the volume of the concerned brain region obtained from the follow-up brain image, Vold is the volume of the concerned brain region obtained from the baseline brain image.

At step 109, the system automatically determines a regularized estimate $r_{reg}$ of the volume change rate for each concerned brain region by using a shrinkage estimator of the volume change rate, which advantageously enables to trade lower variance for increased bias by "shrinking" extreme swings towards the mean. Preferentially, said regularized estimate $r_{reg}$ is determined using equation (1), i.e.:

$$r_{reg} = (1-\lambda) r + \lambda m ,$$

wherein, in the present specific embodiment,

m is the coefficient for Age obtained from training the reference data model on healthy controls. m corresponds to the mean of the distribution of subject-specific long-term brain volume change estimates;

$\lambda$ is computed analytically by the system from the variance of measurements of said quantitative parameter for each concerned brain region. Optionally, the system automatically sets $\lambda$ in cross-validation by minimizing a regression error metric (such as mean absolute error (MAE)) for each of said concerned brain regions. Alternatively, the system may set $\lambda = 0.5$ for each of the concerned brain regions.

At step 110, the system automatically stores the regularized estimate $r_{reg}$ of the volume change rate for each concerned brain region.

Advantageously, the regularized estimate $r_{reg}$ of the volume change rate obtained according to the claimed method has approximately half the error (as measured by MAE) than the "short-term" approximative volume change rate r. Studies of the Applicant have shown that for AD, $r_{reg}$ on average moved towards the long-term estimator for all brain regions. For instance, in case of AD and for the ventricles, the mean absolute error was reduced from 4.5% to 2.2% by the regularized estimate $r_{reg}$, bringing the estimation error below the long-term hypertrophy rate estimate (4.3% annual volume change). Advantageously, the determination of the regularized estimate $r_{reg}$ according to the claimed method enables to use the latter to distinguish between artefactual variation (apparent volume change) and actual ventricular enlargement, although other regions still have estimation error above an expected annual change.

Figure 2 illustrates a system 200 for determining, preferentially automatically, a change rate of a quantitative parameter measured for a biological object of a subject. The system comprises:

- a device 201 for measuring a quantitative parameter for said biological object of a subject, said device being for instance an MRI apparatus configured for acquiring quantitative maps for the biological object, e.g. brain images of a subject;
- a database 202 or memory for storing data required for determining the change rate of said quantitative parameter, said database 202 being for instance connected to the device 201 or part of the latter;
- a processing unit 203 configured for processing the data required for determining said change rate, said processing unit 203 being preferentially connected to the device 201 and to the database 202;
- optionally a display 204 for displaying the determined change rate, said display 204 being connected to the processing unit 203;

wherein the system 200 according to the invention is configured for performing the steps of the previously described method for determining the regularized estimate $r_{reg}$ of the quantitative parameter change rate.

Finally, the advantages of the present invention compared to existing techniques can be summarized as follows when considering the specific case of atrophy rate measurement:

- the mixed effects model uses preferentially routine morphometry data that can be obtained rapidly directly from an MRI scanner, rather than having to post-process data using a research software;
- it reduces estimation error in the determination of the volume change rate considerably compared to existing methods that determine the short-term volume change rate directly;
- it only requires two measurements for providing results with a satisfying error. In particular, the described technique provides an improved estimation of atrophy rates by leveraging already existing data in a new way.

**Claims**

1. A method (100) for determining a regularized estimate of a change rate of a quantitative parameter measured for a biological object of a new subject, the method comprising:

    - using a device (201) for measuring (105), for said new subject, said quantitative parameter at a first time T1 and at a second time T2 within a short period of time, wherein the value of the quantitative parameter measured at the first time T1 is denoted Q1 and the value of the quantitative parameter measured at the second time T2 is denoted Q2 (102);
    - using a processing unit (203) for determining (108) an approximative change rate r of the measured quantitative parameter in function of Q2, Q1, and an interval of time d separating the measurement of Q1 from the measurement of Q2; the method being **characterized in that** it automatically determines (109), using the processing unit (203), the regularized estimate $r_{reg}$ from

$$r_{reg} = (1-\lambda)\, r \;+\; \lambda m$$

    wherein

    m is a mean value for said change rate of the quantitative parameter obtained by applying a reference data model to values of said quantitative parameters obtained by measurements of the latter for said biological object of healthy subjects, wherein for each healthy subject, values of the quantitative parameter have been obtained by making a series of said measurements over a long period of time with respect to said short period of time; and
    $\lambda$ is computed analytically using an estimate of the variance of the values of the quantitative parameters measured for said healthy subjects.

2. The method of claim 1, wherein r = 365*(Q2-Q1)/(d*Q1), wherein the interval of time d is given in days.

3. The method of claim 1 or 2, wherein the reference data model is a log-linear model of the quantitative parameter distributions represented as a function of

    - a constant,
    - a fixed effect for the age of a considered healthy subject,
    - a fixed effect for the sex of said considered healthy subject,
    - a random intercept and a random slope for each subject.

4. The method according to one of the claims 1 to 3, wherein said quantitative parameter is a brain region volume and said biological object is a brain.

5. The method according to claim 4, wherein the reference data model is given by:

$$\texttt{log(volume) ~ 1 + Age + Sex + (1 + Age | Subject)}$$

    wherein

volume is the brain region volume;

Age is the age of the healthy subject at the time of a scan configured for acquiring a subject brain image for measuring said brain region volume;

Sex is the biological sex of the healthy subject;

Subject is a categorical variable uniquely identifying the healthy subject.

6. The method according to one of the claims 4 or 5, wherein longitudinal brain images are acquired (101) for each healthy subject.

7. The method according to claim 6, wherein T1-weighted images are acquired for each healthy subject using an MPRAGE MRI sequence acquisition.

8. The method according to one of the claims 4 to 7, comprising registering (102) the images of each healthy subject to their first time point using preferentially an affine registration.

9. The method according to one of the claims 4 to 8, comprising segmenting (103) each brain image of each healthy subject into different brain regions for determining a set of longitudinal brain region volumes for each healthy subject.

10. The method according to claim 9, comprising training (104) the reference data model onto the longitudinal brain region volumes.

11. System (200) for determining a regularized estimate of a change rate of a quantitative parameter measured for a biological object of a new subject, the system comprising:

- a device (201) for measuring the quantitative parameter for said biological object;
- a database (202) for storing data required for determining a change rate of said quantitative parameter;
- a processing unit (203) configured for processing the data required for determining said change rate;

wherein the system (200) is configured for performing the steps of the method according to one of the claims 1 to 10 in order to determine said regularized estimate.

**Patentansprüche**

1. Verfahren (100) zum Bestimmen einer regularisierten Schätzung einer Änderungsrate eines quantitativen Parameters, der für ein biologisches Objekt eines neuen Probanden gemessen wird, wobei das Verfahren Folgendes umfasst:

- Verwenden einer Vorrichtung (201) zum Messen (105) des quantitativen Parameters für den neuen Probanden zu einem ersten Zeitpunkt T1 und zu einem zweiten Zeitpunkt T2 innerhalb einer kurzen Zeitspanne, wobei der Wert des quantitativen Parameters, der zum ersten Zeitpunkt T1 gemessen wird, als Q1 bezeichnet wird und der Wert des quantitativen Parameters, der zum zweiten Zeitpunkt T2 gemessen wird, als Q2 (102) bezeichnet wird;
- Verwenden einer Verarbeitungseinheit (203) zum Bestimmen (108) einer annähernden Änderungsrate r des gemessenen quantitativen Parameters in Abhängigkeit von Q2, Q1 und einem Zeitintervall d, das die Messung von Q1 von der Messung von Q2 trennt;

wobei das Verfahren **dadurch gekennzeichnet ist, dass** es unter Verwendung der Verarbeitungseinheit (203) automatisch den regularisierten Schätzwert $r_{reg}$ aus Folgendem bestimmt (109):

$$r_{reg} = (1-\lambda) \ r \ + \ \lambda m$$

wobei

m ein Mittelwert für die Änderungsrate des quantitativen Parameters ist, der durch Anwenden eines Referenzdatenmodells auf Werte der quantitativen Parameter erlangt wird, die durch Messungen der letzteren für das biologische Objekt gesunder Probanden erlangt wurden, wobei für jeden gesunden Probanden Werte des quantitativen Parameters erlangt wurden, indem eine Reihe der Messungen über einen langen Zeitraum in Bezug auf den kurzen Zeitraum durchgeführt wurde; und

A analytisch unter Verwendung einer Schätzung der Varianz der Werte der für die gesunden Probanden gemessenen quantitativen Parameter berechnet wird.

2. Verfahren nach Anspruch 1, wobei r = 365* (Q2-Q1)/(d*Q1), wobei das Zeitintervall d in Tagen angegeben ist.

3. Verfahren nach Anspruch 1 oder 2, wobei das Referenzdatenmodell ein log-lineares Modell der quantitativen Parameterverteilungen ist, dargestellt als eine Abhängigkeit

   - von einer Konstante,
   - von einem fixen Effekt für das Alter eines als gesund geltenden Probanden,
   - von einem fixen Effekt für das Geschlecht des als gesund geltenden Probanden,
   - von einem zufälligen Achsenabschnitt und einer zufälligen Steigung für jeden Probanden.

4. Verfahren nach einem der Ansprüche 1 bis 3, wobei der quantitative Parameter ein Gehirnregionen-Volumen ist und das biologische Objekt ein Gehirn ist.

5. Verfahren nach Anspruch 4, wobei das Referenzdatenmodell gegeben ist durch:

log (Volumen) ~ 1 + Alter + Geschlecht + (1 + Alter | Proband) wobei

   Volumen das Gehirnregionen-Volumen ist;
   Alter das Alter des gesunden Probanden zum Zeitpunkt eines Scans ist, der zum Aufnehmen eines Probanden-Gehirnbildes zur Messung des Gehirnregionen-Volumens konfiguriert ist;
   Geschlecht das biologische Geschlecht des gesunden Probanden ist;
   Proband eine kategoriale Variable ist, die den gesunden Probanden eindeutig identifiziert.

6. Verfahren nach einem der Ansprüche 4 oder 5, wobei für jeden gesunden Probanden longitudinale Bilder des Gehirns aufgenommen (101) werden.

7. Verfahren nach Anspruch 6, wobei für jeden gesunden Probanden unter Verwendung einer MPRAGE-MRT-Sequenzerfassung T1-gewichtete Bilder aufgenommen werden.

8. Verfahren nach einem der Ansprüche 4 bis 7, umfassend Registrieren (102) der Bilder jedes gesunden Probanden zu ihrem ersten Zeitpunkt unter Verwendung vorzugsweise einer affinen Registrierung.

9. Verfahren nach einem der Ansprüche 4 bis 8, umfassend Segmentieren (103) jedes Gehirnbildes jedes gesunden Probanden in verschiedene Gehirnregionen zum Bestimmen eines Satzes longitudinaler Gehirnregionen-Volumina für jeden gesunden Probanden.

10. Verfahren nach Anspruch 9, umfassend Trainieren (104) des Referenzdatenmodells auf die longitudinalen Gehirnregionen-Volumina.

11. System (200) zum Bestimmen einer regularisierten Schätzung einer Änderungsrate eines quantitativen Parameters, der für ein biologisches Objekt eines neuen Probanden gemessen wird, wobei das System Folgendes umfasst:

   - eine Vorrichtung (201) zum Messen des quantitativen Parameters für das biologische Objekt;
   - eine Datenbank (202) zum Speichern von Daten, die zum Bestimmen einer Änderungsrate des quantitativen Parameters erforderlich sind;
   - eine Verarbeitungseinheit (203), die dazu konfiguriert ist, die zum Bestimmen der Änderungsrate erforderlichen Daten zu verarbeiten;

wobei das System (200) zum Durchführen der Schritte des Verfahrens nach einem der Ansprüche 1 bis 10 konfiguriert ist, um die regularisierte Schätzung zu bestimmen.

**Revendications**

1. Procédé (100) pour déterminer une estimation régularisée d'une vitesse de changement d'un paramètre quantitatif mesuré pour un objet biologique d'un nouveau sujet, le procédé comprenant :

   - l'utilisation d'un dispositif (201) pour mesurer (105), pour ledit nouveau sujet, ledit paramètre quantitatif à un premier instant T1 et à un second instant T2 sur une courte période de temps, dans lequel la valeur du paramètre quantitatif mesuré au premier instant T1 est notée Q1 et la valeur du paramètre quantitatif mesuré au second instant T2 est notée Q2 (102) ;
   - l'utilisation d'une unité de traitement (203) pour déterminer (108) une vitesse de changement approximative r du paramètre quantitatif mesuré en fonction de Q2, Q1 et d'un intervalle de temps d séparant la mesure de Q1 de la mesure de Q2 ;

   le procédé étant **caractérisé en ce qu'**il détermine automatiquement (109), en utilisant l'unité de traitement (203), l'estimation régularisée $r_{reg}$ à partir de

   $$r_{reg} = (1-\lambda)r + \lambda m$$

   dans lequel

   m est la valeur moyenne de ladite vitesse de changement du paramètre quantitatif obtenue par application d'un modèle de données de référence aux valeurs desdits paramètres quantitatifs obtenues par des mesures de ces derniers pour ledit objet biologique de sujets sains, dans lequel, pour chaque sujet sain, les valeurs du paramètre quantitatif ont été obtenues en effectuant une série desdites mesures sur une longue période de temps par rapport à ladite courte période de temps ; et
   $\lambda$ est calculé analytiquement en utilisant une estimation de la variance des valeurs des paramètres quantitatifs mesurés pour lesdits sujets sains.

2. Procédé selon la revendication 1, dans lequel r = 365*(Q2-Q1)/(d*Q1), dans lequel l'intervalle de temps d est exprimé en jours.

3. Procédé selon la revendication 1 ou 2, dans lequel le modèle de données de référence est un modèle log-linéaire des distributions de paramètres quantitatifs représentées en fonction de

   - une constante,
   - un effet fixe pour l'âge d'un sujet sain considéré,
   - un effet fixe pour le sexe dudit sujet sain considéré,
   - une interception aléatoire et une pente aléatoire pour chaque sujet.

4. Procédé selon l'une des revendications 1 à 3, dans lequel ledit paramètre quantitatif est le volume d'une région cérébrale et ledit objet biologique est un cerveau.

5. Procédé selon la revendication 4, dans lequel le modèle de données de référence est donné par :

   $$\log(\text{volume}) \sim 1 + \text{Âge} + \text{Sexe} + (1 + \text{Âge} \mid \text{Sujet})$$

   dans lequel

   volume est le volume de la région cérébrale ;
   Âge est l'âge du sujet sain au moment d'un scanner configuré pour acquérir une image cérébrale du sujet afin de mesurer ledit volume de la région cérébrale ;
   Sexe est le sexe biologique du sujet sain ;
   Sujet est une variable catégorielle identifiant de manière unique le sujet sain.

6. Procédé selon l'une des revendications 4 ou 5, dans lequel des images cérébrales longitudinales sont acquises (101) pour chaque sujet sain.

7. Procédé selon la revendication 6, dans lequel des images pondérées en T1 sont acquises pour chaque sujet sain en utilisant une acquisition de séquence IRM MPRAGE.

8. Procédé selon l'une des revendications 4 à 7, comprenant le recalage (102) des images de chaque sujet sain à leur premier point temporel, de préférence par recalage affine.

9. Procédé selon l'une des revendications 4 à 8, comprenant la segmentation (103) de chaque image cérébrale de chaque sujet sain en différentes régions cérébrales afin de déterminer un ensemble de volumes longitudinaux de régions cérébrales pour chaque sujet sain.

10. Procédé selon la revendication 9, comprenant l'apprentissage (104) du modèle de données de référence sur les volumes longitudinaux de régions cérébrales.

11. Système (200) pour déterminer une estimation régularisée d'une vitesse de changement d'un paramètre quantitatif mesuré pour un objet biologique d'un nouveau sujet, le système comprenant :

- un dispositif (201) pour mesurer le paramètre quantitatif dudit objet biologique ;
- une base de données (202) pour stocker les données nécessaires à la détermination de la vitesse de changement dudit paramètre quantitatif ;
- une unité de traitement (203) configurée pour traiter les données nécessaires à la détermination de ladite vitesse de changement ;

dans lequel le système (200) est configuré pour réaliser les étapes du procédé selon l'une des revendications 1 à 10 afin de déterminer ladite estimation régularisée.

100

```
┌─────────┐
│   101   │
└─────────┘
     │
     ▼
┌─────────┐
│   102   │
└─────────┘
     │
     ▼
┌─────────┐
│   103   │
└─────────┘
     │
     ▼
┌─────────┐
│   104   │
└─────────┘
     │
     ▼
┌─────────┐
│   105   │
└─────────┘
     │
     ▼
┌─────────┐
│   106   │
└─────────┘
     │
     ▼
┌─────────┐
│   107   │
└─────────┘
     │
     ▼
┌─────────┐
│   108   │
└─────────┘
     │
     ▼
┌─────────┐
│   109   │
└─────────┘
     │
     ▼
┌─────────┐
│   110   │
└─────────┘
```

FIG 1

200

201

202

203

204

FIG 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BLAKE C. JONES et al.** Quantification of multiple-sclerosis-related brain atrophy in two heterogeneous MRI datasets using mixed-effects modeling. *Neuro-Image: Clinical*, 2013, vol. 3, 171-179
- **TEIPEL et al.** Multivariate deformation-based analysis of brain atrophy to predict Alzheimer's disease in mild cognitive impairment. *NeuroImage*, 2007

- **LEDIG et al.** Structural brain imaging in Alzheimer's disease and mild cognitive impairment: biomarker analysis and shared morphometry database. *Sci. Rep*, 2018, vol. 8
- **SCHOTT et al.** Combining short interval MRI in Alzheimer's disease. *J. Neurol.*, 2006, vol. 253, 1147-1153